# EUROPEAN PATENT SPECIFICATION

(11) **EP 1 541 115 B1**
(45) Date of publication and mention of the grant of the patent: **01.06.2011**
(21) Application number: 03764158.6
(22) Date of filing: 09.07.2003
(51) Int. Cl.: A61K 8/04, A61K 8/25, A61Q 1/00, A61Q 1/02, A61Q 1/12, A61Q 19/00, A61Q 1/10

(54) **COSMETIC**
KOSMETIKUM
PRODUIT COSMETIQUE

(30) Priority: 11.07.2002 JP 2002203242
(43) Date of publication of application: 15.06.2005
(73) Proprietor: JGC Catalysts and Chemicals Ltd., Kawasaki-shi Kanagawa (JP)
(72) Inventor: MIYAZAKI, T., Wakamatsu Factory Catalysts Chem., Kitakyushu-shi, Fukuoka 808-0027 (JP); TANAKA, H., Wakamatsu Factory, Catalysts & Chem., Kitakyushu-shi, Fukuoka 808-0027 (JP)
(74) Representative: Westendorp, Michael Oliver
(86) International application number: PCT/JP2003/008755
(87) International publication number: WO 2004/006873

(56) References cited:
- EP-A- 0 543 999
- EP-A- 1 066 818
- JP-A- 2002 160 907
- US-A- 4 775 520
- US-A- 5 846 310
- US-B1- 6 342 193
- DATABASE WPI Week 2002 Derwent Publications Ltd., London, GB; AN 2002-611819 XP002337489 "Spherical porous particle, e.g. for heat insulating material, noise reduction material and cosmetics, contains two types of inorganic microparticle aggregates with different particle diameter, coated with silica layer" & JP 2002 611819 A (SHOKUBAI KASEI KOGYO KK) 4 June 2002 (2002-06-04)
- DATABASE WPI Week 1997 Derwent Publications Ltd., London, GB; AN 1997-161369 XP002337490 "Transparent cosmetics for coevring skin-comprises coated clay mineral with inorganic metal oxide" & JP 09 030917 A (MIYOSHI KASEI YG) 4 February 1997 (1997-02-04)
- STOBER W ET AL: "CONTROLLED GROWTH OF MONODISPERSE SILICA SPHERES IN THE MICRON SIZERANGE" JOURNAL OF COLLOID AND INTERFACE SCIENCE, ACADEMIC PRESS, NEW YORK, NY, US, vol. 26, 1968, pages 62-69, XP000561462 ISSN: 0021-9797

## Description

### TECHNICAL FIELD

The present invention relates to cosmetics comprising spherical, nonporous particles substantially with no pore volume and having a blurring effect.

### BACKGROUND ART

Spherical particles have been used to give smooth tactile impression to cosmetics by making use of rolling of the particles when the particles are applied to human skin. The present applicant proposes cosmetics with particles of inorganic compounds such as spherical titanium oxide blended therein in Japanese Patent Publication No. HEI 4-25208, and also cosmetics with spherical silica particles blended therein in Japanese Patent Publication No. HEI 3-43202.

Further make-up cosmetics with fine and spherical silica gel having a specific particle diameter blended therein is disclosed in Japanese Patent Laid-Open Publication No. SHO 54-140736, and solid type powder ceruse with spherical silicate anhydrate having a specific particle diameter is disclosed in Japanese Patent Laid-Open Publication No. SHO 54-55739. Further cosmetics with colored and spherical particles prepared by enveloping inorganic pigments with an inorganic and porous material for improving a coloring effect of the inorganic pigments blended therein is disclosed in Japanese Patent Laid-Open Publication No. HEI 7-145021.
Further there has also been known a cosmetics with porous silica particles blended therein for giving a sebum absorbing effect or deodorizing effect to the cosmetics.
However, the cosmetics with the spherical particles blended therein can give the smooth tactile impression, but can not give an effect of blurring and making invisible skin defects such as fine wrinkles. In addition, there still remain problems such as that the cosmetic effects provided by the products is rather too strong.

On the other hand, as cosmetics for hiding or blurring defects of human skin such as fine wrinkles and the like, there has been known cosmetics with scarious material blended therein. For instance, WO Publication 92/03119 discloses cosmetics with composite powder having an effect of blurring a base by scattering light with submicron spherical silica particles deposited on a surface of the scarious material. Further WO Publication 99/49834 discloses cosmetics having a coating effect and transparent feelings with inorganic composite powder blended therein, the inorganic composite powder being prepared by laminating two or more types of inorganic oxides having different refractive indexes respectively on a surface of the scarious material with those having higher refractive indexes at lower layers. With the cosmetics with the scarious material blended therein, as naturally expected, it is impossible to obtain the smooth tactile impression such as that provided by the cosmetics with spherical particles blended therein.

The present applicant previously proposed to obtain fine particles of composite oxide having a low refractive index by completely coating a surface of porous fine particle of inorganic oxide with such a material as silica (Refer to Japanese Patent Laid-Open Publication No. HEI 7-133105). With the invention, however, a diameter of obtained particles is small, and the particles do not give sufficient smoothness and smooth tactile impression when used in cosmetics.
The present inventors disclosed, in Japanese Patent Laid-Open Publication No. 2002-160907, the fact that spherical and porous particles each comprising an aggregate of inorganic oxide fine particles with an average particle diameter in a range from 1 to 100 µm comprising fine particles of inorganic oxide having an average diameter in a range from 2 to 250 nm and silica-based layer coating the aggregate have a very low refractive index, and also the fact that a very light, soft, and high extendable foundation can be obtained by using the spherical porous particles as a high grade smoothing filler in cosmetics.

### DISCLOSURE OF INVENTION

The present inventors made strenuous efforts for examining the possibility of cosmetics using particles prepared by coating aggregate of fine particles of inorganic oxide with silica to find that, of the particles as described above, the cosmetics using the spherical and nonporous particles with an especially fine silica-coated layer gives the smooth tactile impression and has the effect of blurring defects of human skin such as fine wrinkles, and complete this invention.
Namely an object of the present invention is to provide cosmetics comprising spherical and nonporous particles substantially with no pore volume and having the blurring effect. More specifically an object of the present invention is to provide cosmetics capable of giving smooth and soft tactile impression and making defects of skin such as fine wrinkles not so visible when applied to skin because the spherical and nonporous particles are included and also capable of suppressing the decorative effect of cosmetics.

The cosmetics according to the present invention includes spherical and nonporous particles by 0.1 to 70% by weight, a liquid component, and solid components other than the spherical and nonporous particles, and is characterized in that the spherical and nonporous particles each comprise aggregate of inorganic oxide particles having an average particle diameter in a range from 1 to 100 µm comprising fine particles of inorganic oxide having an average particle diameter in a range from 2 to 250 nm and silica-based layer coating the aggregate.

The liquid component should preferably be included therein by 10% or more by weight.
The refractive index of the spherical and nonporous particles is preferably in a range from 1.10 to 1.42 and a particle density is preferably in a range from 1 to 2 g/ml.
The thickness (Tₛ) of the silica-based layer is preferably in a range from 0.002 to 25 µm and a ratio of the thickness of the silica-based layer (Tₛ) versus an average particle diameter (P_{D}) of the spherical and nonporous particles ((Tₛ)/(P_{D})) is preferably in a range from 0.001 to 0.25.

The spherical and nonporous particles are preferably those prepared through the following steps (a) to (e):
(a) a step of preparing aggregate of inorganic oxide fine particles by spraying a colloidal solution of inorganic oxide fine particles or, if required, a colloidal solution including a hydrogel of inorganic oxide and/or xerogel into an air stream;
(b) a step of heating the aggregate of inorganic oxide fine particles at a temperature in a range from 150 to 600 °C;
(c) a step of dispersing the aggregate of inorganic oxide fine particles in water and/or an organic solvent;
(d) a step of coating an external surface of the aggregate with a silica-based layer by adding an acid or alkaline aqueous solution and at least one of a silicic acid solution, an organic silicon compound expressed by the chemical formula (1) or a partially hydrolyzed product thereof into the dispersion of the aggregate of inorganic oxide fine particles:

   RₙSi(OR')₄₋ₙ (1)

   wherein R and R' indicate hydrocarbon groups such as an alkyl group, an aryl group, a vinyl group, and acrylic group, and n is an integral number in a range from 0 to 3; and
(e) a step of subjecting the dispersion of the aggregate of inorganic oxide fine particles coated with the silica-based layer to
   (A) hydrothermal processing at a temperature in a range from 50 to 350 °C, or
   (B) separating and drying the inorganic oxide fine particles coated with the silica-based layer, and then heating the inorganic oxide fine particles under atmospheric pressure or in a depressurized state at a temperature in a range from 400 to 1200 °C.

The method of producing cosmetics according to the present invention - comprises a step of obtaining spherical and nonporous particles through the sub steps (a) to (e) and a step of blending the spherical and nonporous particles in a liquid component:
(a) a step of preparing aggregate of inorganic oxide fine particles by spraying a colloidal solution of inorganic oxide fine particles into an air stream;
(b) a step of heating the aggregate of inorganic oxide fine particles at a temperature in a range from 150 to 600 °C;
(c) a step of dispersing the aggregate of inorganic oxide fine particles in water and/or an organic solvent;
(d) a step of coating an external surface of the aggregate with a silica-based layer by adding an acid or alkaline aqueous solution and at least one of a silicic acid solution, an organic silicon compound expressed by the chemical formula (1) or a partially hydrolyzed product thereof into the dispersion of the aggregate of inorganic oxide fine particles:

   RₙSi(OR')₄₋ₙ (1)

   wherein R and R' indicate hydrocarbon groups such as an alkyl group, an aryl group, a vinyl group, and an acrylic group, and n is an integral number in the range from 0 to 3; and
(e) a step of subjecting the dispersion of the aggregate of inorganic oxide fine particles coated with the silica-based layer to
   (A) hydrothermal processing at a temperature in a range from 50 to 350 °C, or
   (B) separating and drying the inorganic oxide fine particles coated with the silica-based layer, and then heating the inorganic oxide fine particles under atmospheric pressure or in a depressurized state at a temperature in a range from 400 to 1200 °C.

In the step (a) above, the aggregate of inorganic oxide fine particles is preferably prepared by spraying a colloidal solution including a hydrogel of inorganic oxide and/or xerogel together with the inorganic oxide fine particles into the air stream.

The cosmetics according to the present invention gives the smooth and soft tactile impression when applied to skin and has the visible effect of blurring defects of skin such as fine wrinkles. Further the cosmetics has the feeling of transparency and suppresses the decorative effect of cosmetics so that natural finishing is insured.

### BEST MODE FOR CARRYING OUT THE INVENTION

### [Spherical and nonporous particles]

Before describing preferred embodiments of the cosmetics according to the present invention, at first the spherical and nonporous particles are described.
The spherical and nonporous particles used in the present invention are prepared by coating aggregate of inorganic oxide fine particles with a silica-based layer, and there are voids between the inorganic oxide fine particles and voids form fine pores.
The average particle diameter of the inorganic oxide fine particles is required to be in the range from 2 to 250 nm, but there is not any specific restriction over the inorganic oxide fine particles, and any of known inorganic oxide fine particles may be used in this invention. More specifically, the inorganic oxide fine particles available for the purpose of this invention include fine particles of silica-based inorganic oxide such as silica, silica alumina, silica zirconia, silica titania. Especially preferable is an oxide sol such as silica sol disclosed by the present applicant in Japanese Patent Laid-Open Publication No. HEI 5-132309 or other documents because the inorganic oxide fine particles are substantially completely spherical. Further fine particles of composite oxide having an organic group disclosed in Japanese Patent Laid-Open Publication No. HEI 10-45403 may advantageously be used for the purpose of this invention. In addition, the composite oxide sol disclosed in Japanese Patent Laid-Open Publication No. HEI 7-133105 applied by the present applicant comprises particles each having voids therein, and is preferably used for obtaining the spherical and nonporous particles having a lower refractive index.

When the average particle diameter of the inorganic oxide fine particles is less than 2 nm, the pore volume provided by voids in the inorganic oxide fine particles are small (0.01 ml/g or below) because the particle diameter is too small, so that the particles are not different from ordinary fine particles with the high density and the effect of dispersing or scattering light is small, so that the effect of blurring defects of skin such as fine wrinkles can not be obtained. Further the refractive index of the spherical and nonporous particles prepared by using the inorganic oxide fine particles may sometimes be over 1.42 giving little feeling of transparency, so that the cosmetics with such particles blended therein can hardly give the natural feeling (the effect of suppressing the decorative effect provided by the cosmetics). When the average particle diameter is more than 250 nm, the pore volume increases, but the bonding force between fine particles is weak, so that it is difficult to obtain the aggregate of inorganic oxide fine particles, and therefore sometimes the spherical and nonporous particles capable of achieving the effect of the present invention can not be obtained. The preferable average particle diameter of the inorganic oxide fine particles is in the range from 5 to 100 nm.

The average particle diameter of the aggregate of inorganic oxide fine particles is preferably in the range from 1 to 100 µm, and further preferably in the range from 2 to 50 µm. When the average particle diameter is more than 100 µm, sometimes distribution of average particle diameter is not uniform, and a percentage of particles having irregular forms and low strength increases, which is disadvantageous. It is difficult to obtain aggregate with the average particle diameter of less than 1 µm.
The aggregate of inorganic oxide fine particles should preferably have the pore volume in the range from 0.01 to 1.2 ml/g. When the pore volume is less than 0.01 cc/g, it is difficult to obtain the effect of lowering the refractive index, and in addition, the effect of blurring the defect of skin such as fine wrinkles can not be obtained because light scatters little. When the pore volume is over 1.2 ml/g, the strength of the aggregate is insufficient, and it is difficult to form a silica-based layer and to obtain the spherical and nonporous particles.

When the pore volume is within the range described above, light entering into inside of the spherical and nonporous particles strikes the inorganic oxide fine particles through an air layer forming a void and is reflected by the inorganic oxide fine particles, and this reflection and transmission are repeated to cause scattering of the light, thus the effect of blurring the defects of skin such as fine wrinkles being obtained. The preferable range of the pore volume is in the range from 0.05 to 0.8 ml/g. The pore volume can be determined by the nitrogen adsorption method (for measuring a quantity of adsorbed nitrogen at the liquid nitrogen temperature and under the relative pressure of 0.6).
Any known method may be employed for producing the aggregate of inorganic oxide fine particles as described above, and the methods available for the purpose of this invention include the micro capsule method, emulsifying method, oil method, spray method and the like. Especially, the method of producing completely spherical particles disclosed in Japanese Patent Publication No. HEI 3-43201, Japanese Patent Publication No. HEI 2-61406 and other related documents makes it possible to obtain aggregate of inorganic oxide fine particles each having a substantially completely spherical form even when the inorganic oxide fine particles used at the initial stage is not spherical, and the production process is not complicated with excellent economical performance. This preferably production method is described hereinafter.

The silica-based layer coating the aggregate of inorganic oxide fine particles includes a layer made from only silica (silica-only layer) and a coating layer comprising silica as a main component and one, two or more of inorganic oxides such as Al₂O₃, B₂O₃, MgO, P₂O₅, Sb₂O₃, MoO₃, ZnO₂, WO₃ and the like. The ratio of silica versus inorganic oxide other than silica each added therein as expressed by the weight ratio MOx/SiO₂, when silica is expressed as SiO₂ and the inorganic oxide other than silica as MOx should preferably be 0.2 or less. When the weight ratio MOx/SiO₂ is over 0.2, it is difficult to form a fine and homogeneous coating layer. The coating effect provided by the coating layer according to the present invention indicates that the obtained spherical and nonporous particles can maintain voids therein and have a low refractive index.

The thickness (Tₛ) of the silica-based layer is preferably in the range from 0.002 to 25 µm, and more preferably in the range from 0.01 to 5 µm. When the thickness (Tₛ) is less than 0.002 µm, the coating layer can hardly showing the coating effect as described above. Namely when actually used, a solvent or the like easily diffuse inside the particles, and sometimes the coating layer can not shown the low refractive index effect, so that the too small thickness (Tₛ) is not preferably. When the thickness (Tₛ) is more than 25 µm, the coating layer is too thick against the particle diameter of spherical and nonporous particles and a volume percentage of voids therein drops, so that the light scattering effect and the low refractive index effect can hardly be expressed. Because of the reasons as described above, a ratio of the thickness (Tₛ) of the silica-based layer versus the average particle diameter (P_{D}) of the spherical and nonporous particles ((Tₛ)/(P_{D})) should preferably be in the range from 0.001 to 0.25, and more preferably in the range from 0.01 to 0.2.
The thickness (Tₛ) of the silica-based layer can be measured by pulverizing the spherical and nonporous particles, photographing cross sections of the pulverized particles with a transmission electron microscope (TEM), measuring thicknesses of coated layer portions of 20 particles, and averaging the values. The average particle diameter of the aggregate and that of the spherical and nonporous particles can be measured by photographing 500 particles with a scanning electron microscope (SEM), measuring the particle diameters of 500 particles, and averaging the measured values.

There is no specific restriction over the method of forming the silica-based layer as described above so long as the coating layer with the thickness in the range as described above can be formed. As the method of forming a fine and homogeneous coating layer, it is preferable to employ, for instance, the method in which a prespecified quantity of a silicic acid solution obtained by dealkalizing an alkali metal salt of silica (water glass) is added in a dispersion of the aggregate of inorganic oxide fine particles and at the same time alkali is added to have the silicic acid solution deposited on an external surface of the aggregate, or the method in which a hydrolyzable organic silicon compound is hydrolyzed with an acid or alkaline catalyst to have the hydrolysate deposited on an external surface of the aggregate. If necessary, it is possible to form the silica-based coating layer including silica as a main component and an inorganic oxide other than silica by adding a precursor inorganic compound salt of the inorganic oxide other than silica together with a silicic acid solution, or an organic silicon compound.

The aggregate of inorganic oxide fine particles may include a dry gel originated from a hydrogel obtained by neutralizing or hydrolyzing the precursor inorganic compound salt of the inorganic oxide fine particles and/or xerogel (sometimes called as gel components hereinafter). The gel components include, for instance, aerogil which is a xerogel of silica obtained by subjecting silicon tetrachloride to gas phase pyrolysis or white carbon which is a xerogel of silica obtained by heating and sintering a silica hydrogel obtained by hydrolyzing a silicate salt.
The average particle diameter of the gel components is preferably in the range from 10 to 500 nm. When the average particle diameter is more than 500 nm, strength of the particles becomes lower, which makes it difficult to form a silica-based layer, and when the average particle diameter is less than 10 nm, the effect of increasing voids in the aggregate is not shown sufficiently.

When the gel component is expressed by MO_{G} and the inorganic oxide fine particles by oxide MOₛ, the blending ratio of gel components, namely the weight ratio MO_{G}/Moₛ is preferably in the range from 5/95 to 90/10, and more preferably in the range from 20/80 to 70/30. When the weight ratio MO_{G}/MOₛ is less than 5/95, the effect of increasing voids by using the gel component is shown insufficiently, and when the weight ratio MO_{G}/MOₛ is over 90/10, strength of the particles may become lower.
A refractive index of the spherical and nonporous particles is preferably in the range from 1.10 to 1.42 and more preferably in the range from 1.20 to 1.40. It is difficult to obtain spherical and nonporous particles with the refractive index of less than 1.10, and when the refractive index is over 1.42, the transparency of the spherical and nonporous particle itself becomes lower, so that the cosmetics obtained by blending the spherical and nonporous particles therein lacks the transparency, and the decorative effect of the cosmetics becomes excessive (with the natural feeling lost). A refractive index of the spherical and porous particles is obtained by measuring the particle density according to JIS Z 8807 at first and then calculating the refractive index from the particle density.

The particle density of spherical and nonporous particles is preferably in the range from 1 to 2 g/ml. If is difficult to obtain spherical and nonporous particles with the particle density of less than 1 g/ml and when the particle density is over 2 g/ml, the transparency of the spherical and nonporous particle itself becomes lower, and the cosmetics obtained by blending the spherical and nonporous particles therein lacks the transparency and the decorative effect becomes excessive.

### [Preparation of spherical and nonporous particles]

Preferable production method of the spherical and nonporous particles described above will be described step by step below.

### Step (a) Preparation of aggregate of inorganic oxide fine particles

Aggregate of inorganic oxide fine particles is prepared by spraying a colloidal solution of the inorganic oxide fine particles or, if required, the colloidal solution including the hydrogel and/or xerogel into an air stream.
As the colloidal solution, a sol prepared by dispersing fine particles of inorganic oxide such as silica, silica alumina, silica zirconia, silica titania in water or an organic solvent may be used. The concentration of the colloidal solution is preferably 5 to 60% by weight, and more preferably in the range from 10 to 50% by weight whether or not the gel components are included in the sol. When the concentration of the colloidal solution is less than 5% by weight, it is difficult to obtain aggregate thereof, and even if the aggregate is obtained, it is difficult to obtain large particles with the diameter in the range from 1 to 100 µm, which is disadvantageous. When the concentration of the colloidal solution is over 60% by weight, the colloidal solution is instable, and it is difficult to obtain spherical aggregate. Further it is impossible to continuously perform the spray drying described hereinafter, so that the yield of aggregate becomes lower.

There is no specific restriction over the method of spray drying the colloidal solution so long as the aggregate as described above can be obtained, and any known method such as a rotary disk method, pressurized nozzle method, two-fluid nozzle method may be employed.
The drying temperature employed in this step varies according to the concentration of the colloidal solution, a processing rate and other factors, but the temperature is preferably in the range from 40 to 150 °C and more preferably in the range from 50 to 120 °C. When the drying temperature is less than 40 °C, drying can not be performed sufficiently, and the colloidal solution is easily deposited on a wall of the spray drying device with the yield lowered, and when the drying temperature is over 150 °C, the drying rate is too high, and sometimes particles each having an apple-like recesses, or donut-like particles are obtained, and aggregate with a substantially completely spherical form can hardly be obtained.

### Step (b) Processing for heating the aggregate of inorganic oxide fine particles

The aggregate of inorganic oxide fine particles obtained in step (a) is heated at a temperature in the range from 150 to 600 °C for strengthening a bonding force between the inorganic oxide fine particles or with the gel components. When the temperature employed in this heating process is less than 150 °C, the effect of strengthening the bonding force is not recognized, and when the temperature employed in the heating process is over 600 °C, the aggregate of inorganic oxide fine particles may shrink, and in that case, voids in the spherical and nonporous particles finally obtained become smaller, which is disadvantageous.

### Step (c) Preparation of a dispersion of the aggregate

The aggregate of inorganic oxide fine particles obtained in step (b) is dispersed in water and/or an organic solvent to prepare a dispersion thereof.
As the organic solvent, monovalent alcohol such as ethanol, propanol, butanol or polyvalent alcohol such as ethylene glycol may be used.
The concentration of the dispersion as converted to that of the oxide of the aggregate is preferably in the range from 0.1 to 40% by weight, and more preferably in the range from 0.5 to 20%. When the concentration is less than 0.1% by weight, the productivity is low, and it is difficult to obtain spherical and nonporous particles with large pore volume. When the concentration is over 40% by weight, the aggregates themselves aggregate in the next step (d), which is disadvantageous.

### Step (d) Formation of a silica-based layer

In step (d), an acid or alkaline aqueous solution, an organic silicon compound expressed by the following chemical formula (1) and/or a partially hydrolyzed product thereof are added in the dispersion of the aggregate to cover an external surface of the aggregate with a silica-based layer:

RₙSi(OR')₄₋ₙ (1)

wherein R and R' indicate hydrocarbon groups such as an alkyl group, an aryl group, a vinyl group, and an acrylic group, and n is an integral number in a range from 0 to 3.
The organic silicon compounds available for the purpose of this invention include, but not limited to, tetramethoxysilane, tetraethoxysilane, tetraisopropoxysilane, methyltrimethoxysilane, dimethyldimethoxysilane, phenyltrimethoxysilane, diphenyldimethoxysilane, methytriethoxysilane, dimethyldiethoxysilane, phenyltriethoxysilane, diphenyldiethoxysilane, isobuthyltrimethoxysilane, vinyltrimethoxysilane, vinyltriethoxysilane, vinyl-tris-(β-methoxy)silane, 3,3,3-trifluoropropyl trimethoxysilane, methyl-3,3,3-trifluoropropyl dimethoxysilane, β-(3,4-epoxycyclohexyl)ethyltrimethoxysilane, γ-glycidoxytripropyl trimethoxysilane, γ-glycidoxypropyl methyldiethoxysilane, γ-glycidoxypropyl triethoxysilane, γ-methacryloxypropyl methyldimethoxysilane, γ-methacryloxypropyl trimethoxysilane, γ-methacryloxypropyl methyldiethoxysilane, γ-methacryloxypropyl triethoxysilane, N-β (aminoethyl) γ-aminopropylmethyl dimethoxysilane, N-β (aminoethyl) γ-aminopropyl trimethoxysilane, N-β (aminoethyl) γ-aminopropyl, triethoxysilane, γ-aminopropyl trimethoxysilane, γ-aminopropyl triethoxysilane, N-phenyl-γ-aminopropyl trimethoxysilane, γ-mercaptopropyl trimethoxysilane, trimethylsilanole, methyltrichlorosilane, methyldichlorosilane, dimethyldichlorosilane, trimethylchlorosilane, phenyltrichlorosilane, diphenyldichlorosilane, vinyltrichlorosilane, trimethylbromosilane, or diethylsilane.

In the case where the spherical and nonporous particles are dispersed in an organic solvent or in any oil component for preparing cosmetics, when a compound with n in the range from 1 to 3 among the organic silicon compounds enlisted above or a compound including a fluorine-substituted alkyl group is used, the dispersibility in the organic solvent is high, and spherical and nonporous particles with high affinity with an organic resin can be obtained.
Although a compound with n of 0 (zero) among the organic silicon compounds enlisted above can be used as it is, as a compound with the n in the range from 1 to 3 has low hydrophilicity, it is preferable to previously hydrolyze the compound so that the compound can homogeneously be mixed in the reaction system. For hydrolysis, any known method for hydrolyzing these organic silicon compounds may be employed. When any basic one such as a hydride of an alkali metal, ammonia water, or amine is used as a catalyst for hydrolysis, the reaction product may be converted to an acidic solution by removing the basic catalyst after hydrolysis. Further when an hydrolyte is prepared by using an acidic catalyst such as an organic acid or an inorganic acid, it is preferable to remove the acidic catalyst with an ion exchange resin or the like after the hydrolysis. The obtained hydrolyte of the organic silicon compound is preferably used in the state of an aqueous solution thereof. The aqueous solution as used herein indicates a solution in which the hydrolyte does not show white turbidity as a gel but is transparent.

In addition to the organic silicon compounds enlisted above, an silicic acid solution obtained by dealkalizing an alkali metal salt of silica (water glass) may be used. When a dispersant for the aggregate particles is only water or when a percentage of water in the organic solvent is high, the coating process can be carried out with the silica sol or the silicic acid solution as described above, and in that case, a prespecified quantity of silicic acid solution is added, and at the same time alkali is added to have the silica sol (or a silicate compound dissolved by the alkali) or the silicic acid solution deposited on an external surface of the aggregate particles.
As the silicic acid solution, a silicic acid solution with the alkali removed therefrom by processing an alkali silicate solution with a cation exchange resin may be used, and an acidic silicic acid solution with the pH in the range from 2 to 4 and having the SiO₂ concentration of about 7% by weight or below is preferable.

It is also possible to form a silica-based layer comprising silica and an inorganic oxide other than silica by adding a precursor metal salt of the inorganic oxide other than silica described above, together with any of the organic silicon compounds enlisted above, a partially hydrolyzed product thereof and/or a silicic acid solution.
As a material of the inorganic oxide other than silica, preferably an alkali-soluble inorganic compound is to be used, and the inorganic compounds as described above include, but not limited to, an alkali metal salt or an alkali earth metal salt of the metal or non-metal oxoacid described above, ammonium salt, or tetra ammonium salt, and more specifically it is preferably to use sodium aluminate, sodium tetraborate, sodium aluminosilicate, sodium phosphate and the like.

When the average particle diameter of and void ratio in the aggregate of inorganic oxide fine particles are taken into considerations, quantities of silica source and that containing the inorganic compound salt other than silica should preferably be adjusted so that (Tₛ)/(P_{D}) described above is in the range from 0.001 to 0.25 and the thickness (Tₛ) of the silica-based layer is in the range from 0.002 to 25 µm.
For instance, a quantity of silica (or silica and inorganic oxide other than silica) required for forming a coating layer with the thickness of 2 µm on an external surface of 100 g inorganic oxide fine particle aggregate with the average particle diameter of 100 µm and the pore volume of 0.3 ml/g is about 12 grams, and it is required to add a silica source or an inorganic compound salt other than silica corresponding to the quantity above. It is to be noted that the density of the coating layer is the same as that of silica (d = 2.2) in the description above because a content of the inorganic oxide other than silica is very small.
The dispersion of the aggregate of inorganic oxide fine particles coated with the silica-based layer can be washed by any of known washing methods such as ultrafiltration. In this case, ultrafiltration may be carried out after alkali metal ions, alkali earth metal ions, ammonium ions and the like are removed with an ion exchange resin or the like.

### Step (e)

A dispersion of the inorganic oxide fine particle aggregate coated with the silica-based layer obtained in step (d) above is subjected to (A) hydrothermal processing at a temperature from 50 to 350 °C or (B) processing for separating the inorganic oxide fine particles coated with the silica-based layer, and then the particles are dried, heated under the atmospheric pressure or in the depressurized state at a temperature from 400 to 1200 °C, thus the silica-based layer coating the aggregate of inorganic oxide fine particles being made finer. Namely by reducing or eliminating pores in the silica-based layer, a solvent and/or gas remains in the internal voids of the spherical and nonporous particles.

The hydrothermal processing (A) is carried out by adding an alkali aqueous solution according to the necessity to adjust pH of the dispersion of the inorganic oxide fine particle aggregate coated with the silica-based layer to 8 to 13 and then heating the dispersion. The temperature employed in the heating processing is preferably in the range from 100 to 300 °C. For carrying out the processing for heating, it is allowable that the concentration of the dispersion is previously diluted or condensed. Further, after this step, the dispersion having been subjected to the hydrothermal processing may be washed, and finally particles are filtered off and separated from the dispersion having been subjected to the hydrothermal processing and dried to obtain first spherical and nonporous particles. As the silica-based layer is made finer in the first spherical and nonporous particles, the pore volume is substantially zero even when the volume is measured by the nitrogen adsorption method (by measuring the nitrogen adsorption rate at the liquid nitrogen temperature and under the relative pressure of 0.6). For the reasons described above, the first spherical and nonporous particles used in this invention have fine pores therein, but it may be said that the particles are substantially nonporous (less than 0.02 ml/g) and have a low refractive index.

In the processing (B), after the inorganic oxide fine particles coated with the silica-based layer are separated and dried and also the dispersion medium in the particles is removed, second spherical and nonporous particles are obtained by heating (or sintering) the particles under the atmospheric pressure or in the depressurized state at a temperature from 400 to 1200 °C. In this case, it may be said that fine pores in the silica-based layer disappear or become finer due to the heating processing under a high temperature and the particles are substantially nonporous and have a low refractive index although the fine pore still remains therein in the same sense as that in the case of the first spherical and nonporous particles. When the temperature employed in the heating processing is less than 400 °C, it is impossible to completely block out the pores in the silica-based layer for making the silica-based layer finer. On the other hand, when the temperature employed in the heating processing is over 1200 °C, the spherical and nonporous particles easily fuse to each other, so that the spherical form is hardly maintained.
As no dispersion medium is present in voids of the second spherical and nonporous particles, the refractive index of the particles is very low. Therefore, when the cosmetics obtained by blending the second spherical and nonporous particles therein is used, not only the blurring effect as described above but also the transparency are obtained, which insures natural feelings.

### [Cosmetics]

A blending ratio of the spherical and nonporous particles in the cosmetics according to the present invention is preferably in the range from 0.1 to 70% by weight, and more preferably in the range from 1 to 40% by weight. When the blending ratio of spherical and nonporous particles is less than 0.1% by weight, the effects of providing smoothness and blurring defects of skin and transparency, which should originally be obtained by blending the spherical and nonporous particles, are not obtained, when the blending ratio is over 70 weight%, the coloring characteristics, oily feeling and the like originally required to cosmetics may be spoiled.

The transparency of the cosmetics is affected by a quantity of water blended therein, a type of oil component contained therein, and other factors. For instance, when the spherical and nonporous particles with the refractive index of 1.39 is blended in a silicone-based oil with the refractive index of 1.39, cosmetics with high transparency can be obtained. Namely as a difference between a refractive index the liquid component and that of the spherical and nonporous particles is smaller, the transparency becomes higher. On the contrary, when the deference in the refractive index is larger, the blurring effect as that provided by a frosted glass sheet is obtained. For the reasons as described above, the difference in the refractive index between the spherical and nonporous particles and other components blended in cosmetics is preferably not more than 0.4, and more preferably not more than 0.2, and most preferably not more than 0.01 for obtaining cosmetics with high transparency.
When the spherical and nonporous particles according to the present invention are blended in cosmetics, the surface of the particles may be subjected to the processing with silicone, a fluorine compound, or metal soap.

The cosmetics according to the present invention include at least one of the components which are generally included in ordinary cosmetics such as, fats such as olive oil, colza oil, beef tallow; waxes such as carnauwa wax, kandelila wax, yellow beeswax; hydrocarbons such as paraffin, squalane, synthetic or botanical squalane, α-olefin oligomer, microcrystalline wax, pentane, hexane; fatty acids such as stearic acid, oleic acid, α-hydroxy acid; alcohols such as isostearic alcohol, octyl dodecanole, lauryl alcohol, ethanole, isopropanol, butyl alcohol, myristic alcohol, cetyl alcohol, stearic alcohol, behenic alcohol, alkyl glyceric ethers; esters such as isopropyl myristate, isopropyl palmitate, ethyl stearate, ethyl oleate, cetyl laurate, decyl oleate; multivalent alcohols such as ethylene glycol, triethylene glycol, polyethylene glycol, propylene glycol, glycerin, diglycerin; sugars such as sorbitol, glucose, sucrose, trehalose; silicone oils such as methyl polysiloxane, methyl polyhydrogen siloxane, methylphenyl silicon oil, various denatured silicone oils, cyclic dimethyl silicone oil; fluorine oils such as perfluoropolyether; various types of macro molecules such as gum arabic, carrageenan, agar, xanthan gum, gelatin, alginic acid, guar gum, albumin, pullulan, carboxyvinyl polymer, cellulose and derivatives thereof, polyacrylic amide, sodium polyacrylate, polyvinyl alcohol; various types of anion-, cation-, or non-ionic surface surfactants; extracts from animals and plants; amino acids and peptides; vitamins; ultraviolet rays protective agents such as those based on cinnamates such as paramethoxy octyl cinnamate, salicylic acid, ester benzoate, urocanic acid, benzophenone, and parabenzophenone; pesticides and corrosion-preventive agents: antioxidants; denatured or not-denatured clay minerals; solvents such as butyl acetate, acetone, toluene; titanium oxide, zinc oxide, aluminum oxide, aluminum hydrate, colcothar, yellow ferric oxide, black ferric oxide, cerium oxide, zirconium oxide, silica, mica, talc, sericite, boron nitrate, barium sulfate, mica titanium having pearl-like glaze, and composites thereof each having various particle diameters, particle diameter distribution, and forms; various types of organic pigments and dyes; water; and aroma chemicals. The inorganic compounds such as titanium oxide, zinc oxide may be subjected to the surface processing with silicon, fluorine, metal soap, and the like.

Further the cosmetics according to the present invention may include methyl polyacrylate, nylon, silicone resin, silicone rubber, polyethylene, polyester, polyurethane and the like as resin particles.
Further the cosmetics according to the present invention may include liquids extracted from plants such as albumin, asparaginic acid, vitamin C, sodium ascorbate, ascorbate-phosphate ester magnesium, ascrobyl diparmate, glucoside ascorbate, other derivatives from ascorbic acid, placenta extract, sulfur, fat-soluble extract from licorice root, mulberry extract, linoic acid, linoleic acid, lactic acid, tranexamic acid and the like as active ingredients having the whitening effect.

The cosmetics according to the present invention may include, as the active ingredients having the effect of improving skin roughness, active ingredients having the anti-aging effect such as vitamin C, carotenoid, flavonoid, tannin, café-derivatives, lignin, saponin, retinoic acid and analogous compounds having the structure like retinoic acid, N-acetyl glucosamine, α-hydroxylic acid; polyvalent alcohols such as glycerin, propylene alcohol, 1,3-butylene alcohol; sugars such as mixed isomerized sugars, trehalose, pullulan; biomacromolecules such as sodium hyalunorate, collagen, elastin, chitin chitosan, chondroitin sodium sulfate; and other materials such as amino acid, betaine, ceramide, sphingolipid, cholesterol and derivatives thereof, ε-pronic acid aminate, glycryrrhitinic acid, and various types of vitamin.

The cosmetics according to the present invention can include any of ingredients described in the Reference manual for raw materials of cosmetics (published by Yakuji Nippou K. K., December 25, 2000) or Standard of ingredients for ingredients of cosmetics (Audited by Examination Div. Pharmaceuticals and Chemicals Dep., Ministry of Health and Welfare, published by Yakuji Nippou K. K., April 18, 1997), but ingredients of the cosmetics according to the present invention are not limited to those described above.
The cosmetics according to the present invention can be produced by employing any normal method, and can be used with any form including powdery, cake-like, pencil-like, stick-like, and creamy form, and more specifically can be used as foundation, cream, emulsion, eye shadow, substrate for cosmetics, eye enamel, eye liner, mascara, lip sticks, pack and the like.

The cosmetics according to the present invention can provide the blurring effect, transparency, and smoky feeling such as that provided by a frost glass sheet when the spherical and nonporous particles are blended therein even in the format of not only liquid or creamy form, but also powdery or cake-like form. The liquid components includes, but not limited to, water, higher aliphatic alcohols, oil contents such as ester oil, silicone oil, silicone-based gel, alcohols such as ethyl alcohol, propylene glycol, sorbitol, glycerin, mucopolysaccharide, collagens, PCA salt, moisturizing agents such as lactate, various types of non-ion-, cation-, anion-based or amphoteric surface surfactants, gum arabic, xanthan gum, solvents such as polyvinyl pyrrolidone, ethyl cellulose, carboxymethyl cellulose, carboxyvinyl monomer, ethyl acetate, acetone, toluene, pharmaceuticals, ultraviolet ray absorbents, and fragrant materials.
For obtaining higher transparency or excellent blurring effect like that provided a frosty glass sheet, a contents of the liquid content should preferably be in the range from 10 to 99.9% by weight, and more preferably in the range from 20 to 99% by weight.

### EXAMPLES

### [Production Example 1]

1000 grams of silica sol (produced by Catalysts & Chemicals Industries Co., Ltd., Cataloid S-20L with the average particle diameter of 12 nm and concentration of 20% by weight) was used as a colloidal solution of spherical and nonporous particles and sprayed and dried in a drying air stream with the temperature of 105 °C using a two-fluid nozzle and by feeding the sol at the flow rate of 5 kg/hr to one of the two nozzles and also feeding air at the flow rate of 10500 L/hr to the other nozzle (air/liquid ratio of 2100. Mach 1.1). The obtained powder was sintered for 5 hours at the temperature of 500 °C to obtain aggregate of inorganic oxide fine particles (A-1). The average particle diameter and pore volume of the aggregate were measured to obtain a result as shown in Table 1.
Then 20 grams of the aggregate (A-1) was dispersed in 500 grams of deionized water with the pH adjusted to 11.5 using ammonia water with the concentration of 28% by weight, and 65 grams of silicic acid solution (SiO₂ with the concentration of 6% by weight) was added in the dispersion over 14 minutes keeping the temperature of the dispersion at 90 °C to prepare a dispersion of aggregate of inorganic oxide fine particles coated with a silica layer (A-2).
Then the dispersion was filled in an autoclave and was subjected to the hydrothermal processing for 10 hours at the temperature of 180 °C and then was cooled, filtered to separate the solid phase off, and dried to obtain the spherical and nonporous particles coated with a silica layer containing a methyl group (A-3). The thickness of the coating layer, average particle diameter, pore volume, and particle density, and the refractive index calculated from the parameters above are as shown in Table 2.

### Measurement of the total optical transmittance and haze

3.6 grams of spherical and nonporous particles (A-3) was added to 36 grams of clear lacker (produced by Endo Kagaku Seisakusho) and was homogeneously dispersed with a ultrasonic dispersing device. This dispersion was applied with a bar coater NO. 18 onto a PET film sheet (produced by Tore Corp., lumilar T-60), and was dried under the room temperature. The thickness of the coating film in this step was about 5 µm. Three sheets of coated PET film were prepared under the completely same conditions. The total optical transmittance and haze were measured at two points on each of the coated PET film sheets with a haze meter (produced by Suga Tester, haze computer), and a result of the measurement is as shown in Table 3.

### [Production Example 2]

60 grams of water and 150 grams of aerosil (produced by Nippon Aerosil (K.K.)with the average particle diameter of 0.05µm) were added in 300 grams of silica sol (produced by CCIC: Cataloid SI-50 with the average particle diameter of 25 nm and the concentration of 50% by weight), and further water was added to the resultant mixed sol to prepare a colloidal solution of inorganic oxide fine particles with the concentration of 20% by weight, and further the colloidal solution was spray-dried and the obtained solid phase was sintered like in Example 1 to obtain aggregate of the inorganic oxide fine particles (B-1). The average particle diameter and the void ration (pore volume) were measured, and a result is as shown in Table 1.

Then 20 grams of the aggregate (B-1) was added to a mixed solvent prepared by adding 400 grams of ammonia water with the concentration of 29% by weight into 1300 grams of deionized water and 1100 grams of ethanol, and 14 grams of tetraethoxysilane (produced by Tama Kagaku Kogyo K. K., ethyl silicate A with the SiO₂ concentration of 28% by weight) as an organic silicon compound was added over 14 minutes in the mixed solvent keeping the temperature of the dispersion at 35 °C. Then this dispersion was filled in an autoclave and was subjected to hydrothermal processing for 10 hours at the temperature of 180 °C and was then cooled and filtered to separate off the solid phase, and the separated solid phase was dried to obtain spherical and nonporous particles coated with a fine silica layer (B-2). The thickness of the coating layer, average particle diameter, pore volume, particle density and the refractive index calculated from the parameters above are as shown in Table 2. Further the total optical transmittance and haze were measured, and a result of the measurement is as shown in Table 3.

### (Comparative Example of Production 1)

The particle density of the aggregate of inorganic oxide fine particles (A-1) obtained during the production process in Production Example 1 and a refractive index of the particles were measured, and a result is shown in Table 2. Further the total optical transmittance and haze were measures, and a result is shown in Table 3.

### (Comparative Example of Production 2)

The particle density of the aggregate of inorganic oxide fine particles (A-2) obtained during the production process in Production Example 1 and a refractive index of the particles were measured, and a result is shown in Table 2. Further the total optical transmittance and haze were measures, and a result is shown in Table 3.

### [Production Example 3]

200 grams of water and 180 grams of aerosil (produced by Nippon Aerosil (K.K.) with the average particle diameter of 0.05 µm) were added in 100 grams of silica sol (produced by CCIC; Cataloid S-20L with the average particle diameter of 12 nm and the concentration of 20% by weight) and further water was added to the resultant mixed sol to prepare a colloidal solution of inorganic oxide fine particles with the concentration of 20% by weight, and further the colloidal solution was spray-dried like in Production Example 1 and further the obtained solid phase was sintered to obtain aggregate of the inorganic oxide fine particles (C-1). The average particle diameter and the void ration (pore volume) were measured, and a result is as shown in Table 1.
Then 20 grams of the aggregate (C-1) was added to a mixed solvent prepared by adding 400 grams of ammonia water with the concentration of 29% by weight into 1300 grams of deionized water and 1100 grams of ethanol, and 7 grams of methyltriethoxysilane (produced by Shinnetsu Kagaku K. K., KBE-13 with the SiO₂ concentration of 34% by weight) as an organic silicon compound was added over 14 minutes in the mixed solvent keeping the temperature of the dispersion at 35 °C. Then this dispersion was filled in an autoclave and was subjected to hydrothermal processing for 10 hours at the temperature of 180 °C and was then cooled and filtered to separate off the solid phase, and the separated solid phase was dried to obtain spherical and nonporous particles coated with a fine silica layer containing a methyl group (C-2). The thickness of the coating layer, average particle diameter, pore volume, particle density and the refractive index calculated from the parameters above are as shown in Table 2. Further the total optical transmittance and haze were measured, and a result of the measurement is as shown in Table 3.

### [Example 1]

The following materials A to E were mixed in the blending ratios (% weight) described in accordance with each material to prepare an emulsion. Materials A were previously heated and dissolved at 80 °C and mixed uniformly. The materials B, C, and D were each mixed uniformly.
The material C was mixed to the materials B and dispersed evenly by making use of a roll mill, and then the materials D were added to the mixture and mixed together uniformly. With stirring the mixture, the materials A were added to emulsify, and after the materials E were added, the resulting solution was cooled down to 35 °C when stirring was stopped and left to stand to prepare the emulsion.

| | | |
|---|---|---|
| A. | Monostearic acid polyoxyethylene sorbitan | 1.0 |
| | Tetra oleic acid polyoxyethylene sorbitol | 1.5 |
| | Monostearic acid glyceryl | 1.5 |
| | Stearic acid | 0.5 |
| | Biphenyl alcohol | 1.0 |
| | Palmitic acid cetyl | 0.5 |
| | Squalene | 5.0 |
| | 2-Ethylhexyl cetyl | 4.0 |
| | Methyl polysiloxane | 0.5 |
| B. | Antiseptic agent | adequate dose |
| | 1,3-Butylene glycol | 10.0 |
| C. | Spherical nonporous particles of Production Example 1 (A-3) | 5.0 |
| D. | Triethanolamine | 0.2 |
| | Deionized water | 38.2 |
| E: | Xanthan gum | 0.1 |
| | Deionized water | 30.0 |

When this emulsion was applied to skin, a membrane was formed thereon giving smooth tactile impression with ground-glass-like and hazy transparency, and the effect of blurring the defects of skin such as fine wrinkles was obtained.

### [Example 2]

An emulsion was prepared by the same method as in Example 1 except that vitamin C was newly used and the materials were blended in the following ratios (% weight).

| | | |
|---|---|---|
| A. | Monostearic acid polyoxyethylene sorbitan | 1.0 |
| | Tetra oleic acid polyoxyethylene sorbitol | 1.5 |
| | Monostearic acid glyceryl | 1.5 |
| | Stearic acid | 0.5 |
| | Biphenyl alcohol | 1.0 |
| | Palmitic acid cetyl | 0.5 |
| | Squalene | 5.0 |
| | 2-Ethylhexyl cetyl | 4.0 |
| | Methyl polysiloxane | 0.5 |
| | Vitamin C | 1.0 |
| B. | Antiseptic agent | adequate dose |
| | 1,3-Butylene glycol | 10.0 |
| C. | Spherical nonporous particles of Production Example 1 (A-3) | 5.0 |
| D. | Triethanolamine | 0.2 |
| | Deionized water | 38.2 |
| E. | Xanthan gum | 0.1 |
| | Deionized water | 30.0 |

When this emulsion was applied to skin, a membrane was formed thereon giving the substantially same smooth tactile impression as that in Example 1, and having ground-glass-like and hazy transparency, and the effect of blurring the defects of skin such as fine wrinkles was obtained.

### [Example 3]

An emulsion was prepared by the same method as in Example 1 except that the spherical nonporous particles (B-2) obtained in Production Example 2 were used.
When this emulsion was applied to skin, a membrane was formed thereon giving the substantially same smooth tactile impression as that in Example 1 with more ground-glass-like and hazy transparency as compared to the emulsion prepared in Example 1, and the effect of blurring the defects of skin such as fine wrinkles was obtained similar to the case in Example 1.

### [Comparative Example 1]

An emulsion was prepared by the same method as that in Example 1 except that the aggregate of inorganic oxide fine particles (A-1) obtained in Production Comparative Example 1 was used.
When this emulsion was applied to skin, the product gave smooth tactile impression substantially similar to that in Example 1, but had less ground-glass-like transparency as compared to the case in Example 1 with little effect of blurring the defects of skin such as fine wrinkles.

### [Comparative Example 2]

An emulsion was prepared by the same method as that in Example 1 except that the aggregate of inorganic oxide fine particles (A-2) obtained in Production Comparative Example 2 was used.
When this emulsion was applied to skin, the product gave smooth tactile impression substantially similar to that in Example 1, but had less ground-glass-like transparency as compared to the case in Example 1 with little effect of blurring the defects of skin such as fine wrinkles.

### [Example 4]

The following materials A, materials B were mixed in the blending ratios (% weight) described in accordance with each material to prepare a powder foundation. The materials B are liquid components.
The materials A were mixed together uniformly while the materials B were well stirred with heating to 70 °C. The materials B were added to the materials A and they were mixed uniformly, and then the mixture was milled and press-formed.

| | | |
|---|---|---|
| A. | Titanium oxide | 7.7 |
| | Colcothar | 0.55 |
| | Yellow ferric oxide | 2.5 |
| | Black ferric oxide | 0.15 |
| | Talc | 10.0 |
| | Mica | 25.1 |
| | Sericite | 35.0 |
| | Spherical nonporous particles (B-2) in Production Example 2 | 8.0 |
| B. | Silicone oil | 4.0 |
| | Squalene | 5.2 |
| | Ester oil | 1.6 |
| | Sorbitan sesquioleat | 0.2 |
| | Aroma chemical | adequate dose |
| | Antiseptic agent | adequate dose |

When this powder foundation was applied to skin, the product gave smooth tactile feeling and natural finishing, and the effect of blurring the defects of skin such as fine wrinkles was obtained.

### [Example 5]

The following material A, materials B were mixed in the blending ratios (% weight) described in accordance with each material to prepare a powder foundation by employing the same method as that in Example 4. Silicone treatment ratio for the materials A should be 2% by weight of each material.

| | | |
|---|---|---|
| A. | silicone-treated titanium oxide | 7.7 |
| | Silicone-treated colcothar | 0.55 |
| | Silicone-treated yellow ferric oxide | 2.5 |
| | Silicone-treated black ferric oxide | 0.15 |
| | Silicone-treated talc | 10.0 |
| | Silicone-treated mica | 18.1 |
| | Silicone-treated barium sulfate | 3.0 |
| | Silicone-treated boron nitride | 3.0 |
| | Silicone-treated pearlescent pigment | 1.0 |
| | Silicone-treated sericite | 35.0 |
| | Silicone-treated sphericalnonporous particles (C-2) of Production Example 3 | 8.0 |
| B. | Silicone oil | 4.0 |
| | Squalene | 5.2 |
| | Ester oil | 1.6 |
| | Sorbitan sesquioleat | 0.2 |
| | Aroma chemical | adequate dose |
| | Antiseptic agent | adequate dose |

When this powder foundation was applied to skin, the product gave smooth tactile feeling and natural finishing, having the effect of blurring the defects of skin such as fine wrinkles, and a powder foundation hardly coming-off due to its volatility was achieved.

### [Comparative Example 3]

A powder foundation was prepared by the same method as that in Example 4 except that the aggregate of inorganic oxide fine particles of Production Comparative Example 1 was used instead of the spherical nonporous particles (B-2) of Production Example 2.
When this powder foundation was applied to skin, the product gave smooth tactile feeling substantially similar to that in Example 4, but had insufficient effect of blurring the defects of skin such as fine wrinkles as compared to the effect in Example 4.

### [Example 6]

A cakey powder foundation was prepared by the same method as that in Example 4 except that the materials were blended in the following ratios.

| | | |
|---|---|---|
| A. | Titanium oxide | 7.7 |
| | Colcothar | 0.55 |
| | Yellow ferric oxide | 2.5 |
| | Black ferric oxide | 0.15 |
| | Talc | 10.0 |
| | Mica | 25.1 |
| | Sericite | 40.8 |
| | Spherical nonporous particles (B-2) of Production Example 2 | 8.0 |
| B. | Silicone oil | 3.0 |
| | Squalene | 1.2 |
| | Ester oil | 0.8 |
| | Sorbitan sesquioleat | 0.2 |
| | Aroma chemical | adequate dose |
| | Antiseptic agent | adequate dose |

When this cakey powder foundation was applied to skin, the product gave smooth tactile feeling substantially similar to that in Example 4, and had the effect of blurring the defects of skin such as fine wrinkles the substantially similar level to the case in Example 4, although having the finishing in which whiteness was visible as compared to the case in Example 4.

### [Example 7]

A powder foundation was prepared by the same method as that in Example 4 except that the materials were blended in the following ratios.

| | | |
|---|---|---|
| A. | Titanium oxide | 7.7 |
| | Colcothar | 0.55 |
| | Yellow ferric oxide | 2.5 |
| | Black ferric oxide | 0.15 |
| | Talc | 10.0 |
| | Mica | 18.1 |
| | Barium sulfate | 3.0 |
| | Boron nitride | 3.0 |
| | Red-tinged pearlescent pigment | 1.0 |
| | Sericite | 35.0 |
| | Spherical nonporous particles (B-2) of Production Example 2 | 8.0 |
| B. | Silicone oil | 4.0 |
| | Squalene | 5.2 |
| | Ester oil | 1.6 |
| | Sorbitan sesquioleat | 0.2 |
| | Aroma chemical | adequate dose |
| | Antiseptic agent | adequate dose |

When this powder foundation was applied to skin, the product gave higher smooth tactile feeling as compared to that in Example 4 and had the effect of blurring the defects of skin such as fine wrinkles while giving moderate luster.

### [Comparative Example 4]

A powder foundation was prepared by the same method as that in Example 4 except that the aggregate of inorganic oxide fine particles (A-2) of Production Comparative Example 2 was used instead of the spherical nonporous particles (B-2) of Production Example 2.
When this powder foundation was applied to skin, the product gave smooth tactile feeling substantially similar to that in Example 4, but did not give natural finishing like in the case in Example 4 with insufficient effect of blurring the defects of skin such as fine wrinkles.

### [Example 8]

The following materials A to D were mixed in the blending ratios (% weight) described in accordance with each material to prepare a liquid foundation. At first, the materials B were dispersed into deionized water and heated to 75 °C. The materials C were well mixed with heating to 80 °C, then the two components were mixed together uniformly. After cooling the mixture, the material D was added therein to prepare a liquid foundation.

| | | |
|---|---|---|
| A. | Deionized water | 63.1 |
| B. | 1,3-butylene glycol | 6.5 |
| | Triethanolamine | 1.5 |
| | Carboxymethyl cellulose | 0.2 |
| | Bentonite | 0.5 |
| | Spherical nonporous particles (A-3) of Production Example 1 | 7.5 |
| | Pigment titanium oxide | 1.0 |
| | Color pigment | adequate dose |
| C. | Stearic acid | 4.0 |
| | Monostearate propylene glycol | 2.0 |
| | Cetostearyl alcohol | 0.2 |
| | Liquid paraffin | 3.0 |
| | Liquefied lanolin | 2.0 |
| | Myristic acid isopropyl | 8.5 |
| | Methylparaben | adequate dose |
| D. | Aroma chemical | adequate dose |

When this liquid foundation was applied to skin, the product gave high transparency and natural finishing, and the effect of blurring the fine wrinkles was observed.

### [Example 9]

The following materials A to D were mixed in the blending ratios (% weight) described in accordance with each material to prepare a liquid foundation. At first, the materials B were dispersed into deionized water and heated to 75 °C. The materials C were well mixed with heating, and then the two components were mixed together uniformly. After cooling the resulting solution, the material D was added therein to prepare a liquid foundation.

| | | |
|---|---|---|
| A. | Deionized water | 60.1 |
| B. | 1,3-butylene glycol | 6.5 |
| | Triethanolamine | 1.5 |
| | Carboxymethyl cellulose | 0.2 |
| | Bentonite | 0.5 |
| | Spherical nonporous particles (A-3) of Production Example 1 | 7.5 |
| | Silicone treated zinc oxide (ZN-310 manufactured by Sumitomo Osaka Cement Co., Ltd) | 2.0 |
| | Monodisperse spherical silica (COSMO 55, 0.55 µm manufactured by CCIC) | 1.0 |
| | Pigment titanium oxide | 1.0 |
| | Color pigment | adequate dose |
| C. | Stearic acid | 4.0 |
| | Monostearate propylene glycol | 2.0 |
| | Cetostearyl alcohol | 0.2 |
| | Liquid paraffin | 3.0 |
| | Liquefied lanolin | 2.0 |
| | Myristic acid isopropyl | 8.5 |
| | Methylparaben | adequate dose |
| D. | Aroma chemical | adequate dose |

When this liquid foundation was applied to skin, the product gave dry-touch feeling without gooey and the high transparency and natural finishing, and the effect of blurring the fine wrinkles with its fine texture was observed.

**Table 1**

| | Inorganic oxide fine particle | | Gel/ silicate blending ratio | Concentration of dispersion liquid | Spray-dry temperature | Average particle diameter | Pore volume |
|---|---|---|---|---|---|---|---|
| | Component | Av. particle diameter (nm) | Mo_{G/}Moₛ | (wt%) | (°C) | (µm) | (ml/g) |
| Example 1 (A-1) | SiO₂ | 12 | - | 20 | 105 | 5 | 0.3 |
| | | | | | | | |
| Example 2 (B-1) | SiO₂ | 25 | 5/5 | 20 | 105 | 5 | 0.8 |
| | | | | | | | |
| Example 3 (C-1) | SiO₂ | 12 | 9/1 | 20 | 105 | 10 | 1.1 |
| | | | | | | | |
| Comparative Example 1 (A-1) | SiO₂ | 12 | - | 20 | 105 | 5 | 0.3 |
| | | | | | | | |
| Comparative Example 2 (A-2) | SiO₂ | 12 | - | 20 | 105 | 5 | 0.3 |

**Table 2**

| | | | | | Spherical nonporous particle | | | |
|---|---|---|---|---|---|---|---|---|
| | Covered layer | | After-Treat | Av. particle diameter | Pore volume | T_{S}/P_{D} | Ref. index | Particle density |
| | Component | Thickness (Ts)(µm) | | (P_{D}) (µm) | (ml/g) | | | (g/ml) |
| Example 1 (A-3) | SiO₂ | 0.15 | A/D | 5.3 | 0 | 0.03 | 1.36 | 1.76 |
| Example 2 (B-2) | SiO₂ | 0.10 | A/D | 5.1 | 0 | 0.01 | 1.32 | 1.55 |
| Example 3 (C-2) | SiO₂ | 0.20 | A/D | 10.1 | 0 | 0.02 | 1.21 | 1.05 |
| Comparative Example 1 (A-1) | _ | _ | _ | 5 | 0.3 | 0 | 1.45 | 2.2 |
| | | | | | | | | |
| Comparative Example 2 (A-2) | SiO₂ | 0.15 | D | 5.3 | 0.27 | 0.03 | 1.43 | 2.1 |

In the "After-treatment" column, "A" indicates the hydrothermal processing, "D" indicates the dry processing.

**Table 3**

| | Total-light transmittance | Haze |
|---|---|---|
| | (%) | (%) |
| Example 1 (A-3) | 91 | 75 |
| | | |
| Example 2 (B-2) | 92 | 80 |
| | | |
| Example 3 (C-2) | 90 | 88 |
| | | |
| Comp. Example 1 (A-1) | 86 | 46 |
| Comp: Example 2 (A-2) | 83 | 51 |

## Claims

1. Cosmetics comprising 0.1 to 70% by weight of spherical and nonporous particles, 10% or more by weight of a liquid component, and the balance of solid components other than the spherical and nonporous particles, wherein each of said spherical and nonporous particles comprises an aggregate of inorganic oxide particles, and a silica-based layer coated thereon, said aggregate having an average particle diameter in a range from 1 to 100 µm and comprising fine particles of inorganic oxide having an average diameter in a range from 2 to 250 nm.

2. Cosmetics according to claim 1, wherein said cosmetics include 20 to 99% by weight of said liquid component.

3. Cosmetics according to claim 1, wherein a refractive index of said spherical and nonporous particles is in a range from 1.10 to 1.42 and a particle density is in a range from 1 to 2 g/ml (g/cc).

4. Cosmetics according to claim 1, wherein said cosmetics includes the spherical and nonporous particles by 1 to 40% by weight.

5. Cosmetics according to claim 1, wherein a thickness (Tₛ) of the silica-based layer is in a range from 0.002 to 25 µm and a ratio of the thickness of the silica-based layer (T_{S}) versus an average particle diameter (P_{D}) of the spherical and nonporous particles ((T_{S})/(P_{D})) is in a range from 0.001 to 0.25.

6. Cosmetics according to claim 1, wherein said spherical and nonporous particles are prepared through the following steps (a) to (e):
(a) a step of preparing aggregate of inorganic oxide fine particles by spraying a colloidal solution of inorganic oxide fine particles or, if required, a colloidal solution including a hydrogel of inorganic oxide and/or xerogel into an air stream;
(b) a step of heating the aggregate of inorganic oxide fine particles at a temperature in a range from 150 to 600 °C;
(c) a step of dispersing the aggregate of inorganic oxide fine particles in water and/or an organic solvent;
(d) a step of coating an external surface of the aggregate with a silica-based layer by adding an acidic or alkaline aqueous solution and at least one of a silicic acid solution, an organic silicon compound expressed by the chemical formula (1) or a partially hydrolyzed product thereof into the dispersion of the aggregate of inorganic oxide fine particles:
RₙSi(OR')₄₋ₙ (1)
wherein R and R' indicate hydrocarbon groups such as an alkyl group, an aryl group, a vinyl group, and acrylic group, and n is an integral number in a range from 0 to 3; and
(e) a step of subjecting the dispersion of the aggregate of inorganic oxide fine particles coated with the silica-based layer to
(A) hydrothermal processing at a temperature in a range from 50 to 350 °C, or
(B) separating and drying the inorganic oxide fine particles coated with the silica-based layer, and then heating the inorganic oxide fine particles under atmospheric pressure or in a depressurized state at a temperature in a range from 400 to 1200 °C.

7. Cosmetics according to claim 1, wherein the aggregate of inorganic oxide fine particles has a pore volume in the range from 0.01 to 1.2 ml/g (cc/g).

8. Cosmetics according to claim 1, wherein the spherical and nonporous particles have pore volume of less than 0.02 ml/g.

9. A method of producing cosmetics according to claim 1 comprising the steps of:
obtaining spherical and nonporous particles through the following sub steps (a) to (e):
(a) a step of preparing aggregate of inorganic oxide fine particles by spraying a colloidal solution of inorganic oxide fine particles into an air stream;
(b) a step of heating the aggregate of inorganic oxide fine particles at a temperature in a range from 150 to 600 °C;
(c) a step of dispersing the aggregate of inorganic oxide fine particles in water and/or an organic solvent;
(d) a step of coating an external surface of the aggregate with a silica-based layer by adding an acid or alkaline aqueous solution and at least one of a silicic acid solution, an organic silicon compound expressed by the chemical formula (1) or a partially hydrolyzed product thereof into the dispersion of the aggregate of inorganic oxide fine particles:
RₙSi(OR')₄₋ₙ (1)
wherein R and R' indicate hydrocarbon groups such as an alkyl group, an aryl group, a vinyl group, and acrylic group, and n is an integral number in the range from 0 to 3; and
(e) a step of subjecting the dispersion of the aggregate of inorganic oxide fine particles coated with the silica-based layer to
(A) hydrothermal processing at a temperature in a range from 50 to 350 °C, or
(B) separating and drying the inorganic oxide fine particles coated with the silica-based layer, and then heating the inorganic oxide fine particles under atmospheric pressure or in a depressurized state at a temperature in a range from 400 to 1200 °C, and blending the spherical and nonporous particles in a liquid component.

10. A method of producing cosmetics according to claim 9, wherein the aggregate of inorganic oxide fine particles is prepared by spraying a colloidal solution including a hydrogel of inorganic oxide and/or xerogel together with the inorganic oxide fine particles into the air stream in the step (a).

## Patentansprüche

1. Kosmetika, umfassend 0,1 bis 70 Gew.-% sphärische und nicht-poröse Teilchen, 10 Gew.-% oder mehr einer flüssigen Komponente und den Rest feste Komponenten, welche verschieden von den sphärischen und nicht-porösen Teilchen sind, wobei jedes der sphärischen und nicht-porösen Teilchen ein Aggregat von anorganischen Oxidteilchen und eine darauf aufgebrachte Schicht auf Siliciumdioxid-Basis umfasst, wobei das Aggregat einen mittleren Teilchendurchmesser in einem Bereich von 1 bis 100 µm aufweist und feine Teilchen von anorganischem Oxid mit einem mittleren Durchmesser in einem Bereich von 2 bis 250 nm umfasst.

2. Kosmetika gemäß Anspruch 1, wobei die Kosmetika 20 bis 99 Gew.-% der flüssigen Komponente enthalten.

3. Kosmetika gemäß Anspruch 1, wobei ein Brechungsindex der sphärischen und nicht-porösen Teilchen in einem Bereich von 1,10 bis 1,42 liegt und eine Teilchendichte in einem Bereich von 1 bis 2 g/mL (g/cm³) liegt.

4. Kosmetika gemäß Anspruch 1, wobei die Kosmetika die sphärischen und nicht-porösen Teilchen in einer Menge von 1 bis 40 Gew.-% enthalten.

5. Kosmetika gemäß Anspruch 1, wobei eine Dicke (T_{S}) der Schicht auf Siliciumdioxid-Basis in einem Bereich von 0,002 bis 25 µm liegt und ein Verhältnis der Dicke der Schicht auf Siliciumdioxid-Basis (T_{S}) zu einem mittleren Teilchendurchmesser (P_{D}) der sphärischen und nicht-porösen Teilchen ((T_{S})/(P_{D})) in einem Bereich von 0,001 bis 0,25 liegt.

6. Kosmetika gemäß Anspruch 1, wobei die sphärischen und nicht-porösen Teilchen durch die folgenden Schritte (a) bis (e) hergestellt werden:
(a) Herstellen eines Aggregats von feinen anorganischen Oxidteilchen durch Sprühen einer kolloidalen Lösung von feinen anorganischen Oxidteilchen oder, falls erforderlich, einer kolloidalen Lösung, enthaltend ein Hydrogel von anorganischem Oxid und/oder Xerogel in einen Luftstrom;
(b) Erwärmen des Aggregats von feinen anorganischen Oxidteilchen bei einer Temperatur in einem Bereich von 150 bis 600°C;
(c) Dispergieren des Aggregats von feinen anorganischen Oxidteilchen in Wasser und/oder einem organischen Lösungsmittel;
(d) Beschichten einer externen Oberfläche des Aggregats mit einer Schicht auf Siliciumdioxid-Basis durch Zugabe einer sauren oder alkalischen wässrigen Lösung und mindestens einer bzw. einem ausgewählt aus einer Kieselsäurelösung, einer organischen Siliciumverbindung, ausgedrückt durch die chemische Formel (1), oder einem partiell hydrolysierten Produkt davon, in die Dispersion des Aggregats von feinen anorganischen Oxidteilchen:
RₙSi(OR')₄₋ₙ (1)
wobei R und R' Kohlenwasserstoffreste wie einen Alkylrest, einen Arylrest, eine Vinylgruppe und Acrylrest bedeuten und n eine ganze Zahl in einem Bereich von 0 bis 3 ist; und
(e) Unterwerfen der Dispersion des Aggregats von feinen anorganischen Oxidteilchen, welche mit der Schicht auf Siliciumdioxid-Basis beschichtet sind,
(A) einer hydrothermischen Verarbeitung bei einer Temperatur in einem Bereich von 50 bis 350°C, oder
(B) einem Abtrennen und Trocknen der feinen anorganischen Oxidteilchen, welche mit der Schicht auf Siliciumdioxid-Basis beschichtet sind, und dann Erwärmen der feinen anorganischen Oxidteilchen unter Atmosphärendruck oder in einem druckverminderten Zustand bei einer Temperatur in einem Bereich von 400 bis 1200°C.

7. Kosmetika gemäß Anspruch 1, wobei das Aggregat von feinen anorganischen Oxidteilchen ein Porenvolumen im Bereich von 0,01 bis 1,2 mL/g (cm³/g) aufweist.

8. Kosmetika gemäß Anspruch 1, wobei die sphärischen und nicht-porösen Teilchen ein Porenvolumen von weniger als 0,02 mL/g aufweisen.

9. Verfahren zur Herstellung von Kosmetika gemäß Anspruch 1, umfassend die Schritte von:
Erhalten von sphärischen und nicht-porösen Teilchen durch die folgenden Teilschritte (a) bis (e):
(a) Herstellen eines Aggregats von feinen anorganischen Oxidteilchen durch Sprühen einer kolloidalen Lösung von feinen anorganischen Oxidteilchen in einen Luftstrom;
(b) Erwärmen des Aggregats von feinen anorganischen Oxidteilchen bei einer Temperatur in einem Bereich von 150 bis 600°C;
(c) Dispergieren des Aggregats von feinen anorganischen Oxidteilchen in Wasser und/oder einem organischen Lösungsmittel;
(d) Beschichten einer externen Oberfläche des Aggregats mit einer Schicht auf Siliciumdioxid-Basis durch Zugabe einer sauren oder alkalischen wässrigen Lösung und mindestens einer bzw. einem ausgewählt aus einer Kieselsäurelösung, einer organischen Siliciumverbindung, ausgedrückt durch die chemische Formel (1), oder einem partiell hydrolysierten Produkt davon, in die Dispersion des Aggregats von feinen anorganischen Oxidteilchen:
RₙSi(OR')₄₋ₙ (1)
wobei R und R' Kohlenwasserstoffreste wie einen Alkylrest, einen Arylrest, eine Vinylgruppe und Acrylrest bedeuten und n eine ganze Zahl im Bereich von 0 bis 3 ist; und
(e) Unterwerfen der Dispersion des Aggregats von feinen anorganischen Oxidteilchen, welche mit der Schicht auf Siliciumdioxid-Basis beschichtet sind,
(A) einer hydrothermischen Verarbeitung bei einer Temperatur in einem Bereich von 50 bis 350°C, oder
(B) einem Abtrennen und Trocknen der feinen anorganischen Oxidteilchen, welche mit der Schicht auf Siliciumdioxid-Basis beschichtet sind, und dann Erwärmen der feinen anorganischen Oxidteilchen unter Atmosphärendruck oder in einem druckverminderten Zustand bei einer Temperatur in einem Bereich von 400 bis 1200°C,
und Mischen der sphärischen und nicht-porösen Teilchen in einer flüssigen Komponente.

10. Verfahren zur Herstellung von Kosmetika gemäß Anspruch 9, wobei das Aggregat von feinen anorganischen Oxidteilchen durch Sprühen einer kolloidalen Lösung, enthaltend ein Hydrogel von anorganischem Oxid und/oder Xerogel zusammen mit den feinen anorganischen Oxidteilchen in den Luftstrom in Schritt (a) hergestellt wird.

## Revendications

1. Produit cosmétique comprenant 0,1 à 70% en poids de particules sphériques et non poreuses, 10% en poids ou plus d'un composant liquide, le reste étant des composants solides autres que les particules sphériques et non poreuses, chacune desdites particules sphériques et non poreuses se composant d'un agrégat de particules inorganiques d'oxyde, et d'une couche à base de silice, revêtue sur celui-ci, ledit agrégat ayant un diamètre moyen de particules situé dans un intervalle allant de 1 à 100 µm et comprenant de fines particules d'oxyde inorganique ayant un diamètre moyen situé dans l'intervalle allant de 2 à 250 nm.

2. Produit cosmétique selon la revendication 1, où ledit produit cosmétique comprend 20 à 99% en poids dudit composant liquide.

3. Produit cosmétique selon la revendication 1, où l'indice de réfraction desdites particules sphériques et non poreuses se situe dans l'intervalle allant de 1,10 à 1,42 et la densité des particules se situe dans un intervalle allant de 1 à 2 g/ml (g/cm³).

4. Produit cosmétique selon la revendication 1, où ledit produit cosmétique comprend les particules sphériques et non poreuses en une quantité allant de 1 à 40% en poids.

5. Produit cosmétique selon la revendication 1, où l'épaisseur (T_{S}) de la couche à base de silice se situe dans un intervalle allant de 0,002 à 25 µm et le rapport de l'épaisseur de la couche à base de silice (T_{S}) au diamètre moyen des particules (P_{D}) des particules sphériques et non poreuses (T_{S}/P_{D}) se situe dans l'intervalle allant de 0,001 à 0,25.

6. Produit cosmétique selon la revendication 1, où lesdites particules sphériques et non poreuses sont préparées selon les étapes (a) à (e) suivantes :
(a) une étape de préparation des agrégats de fines particules d'oxyde inorganique par pulvérisation d'une solution colloïdale de fines particules d'oxyde inorganique ou si nécessaire, d'une solution colloïdale comprenant un hydrogel d'oxyde inorganique et/ou un xérogel dans un courant d'air ;
(b) une étape de chauffage des agrégats de fines particules d'oxyde inorganique à une température située dans l'intervalle allant de 150 à 600°C ;
(c) une étape de dispersion des agrégats des fines particules d'oxyde inorganique dans l'eau et/ou un solvant organique ;
(d) une étape de revêtement de la surface externe des agrégats avec une couche à base de silice, par addition d'une solution aqueuse acide ou alcaline et d'au moins l'un parmi une solution d'un acide silicique, un composé organique du silicium exprimé par la formule chimique (1) ou un produit partiellement hydrolysé de celui-ci, à la dispersion des agrégats des fines particules d'oxyde inorganique :
RₙSi(OR')₄₋ₙ (1)
où R et R' représentent des radicaux hydrocarbonés tels qu'un radical alkyle, un radical aryle, un radical vinyle, un radical acrylique et n est un nombre entier situé dans l'intervalle allant de 0 à 3 ; et
(e) une étape qui consiste à soumettre la dispersion des agrégats de fines particules d'oxyde inorganique revêtus de la couche à base de silice, à
(A) un traitement hydrothermique à une température située dans l'intervalle allant de 50 à 350°C, ou
(B) une séparation et un séchage des fines particules d'oxyde inorganique revêtues de la couche à base de silice, puis chauffage des fines particules d'oxyde inorganique sous pression atmosphérique ou dans un état de dépressurisation, à une température située dans l'intervalle allant de 400 à 1200°C.

7. Produit cosmétique selon la revendication 1, où les agrégats de fines particules d'oxyde inorganique ont un volume des pores situé dans l'intervalle allant de 0,01 à 1,2 ml/g (cm³/g).

8. Produit cosmétique selon la revendication 1, où les particules sphériques et non poreuses ont un volume des pores inférieur à 0,02 ml/g.

9. Procédé de production d'un produit cosmétique selon la revendication 1, comprenant les étapes consistant à :
obtenir les particules sphériques et non poreuses par les sous-étapes (a) à (e) suivantes :
(a) une étape de préparation des agrégats de fines particules d'oxyde inorganique par pulvérisation d'une solution colloïdale de fines particules d'oxyde inorganique dans un courant d'air ;
(b) une étape de chauffage des agrégats de fines particules d'oxyde inorganique à une température située dans l'intervalle allant de 150 à 600°C ;
(c) une étape de dispersion des agrégats des fines particules d'oxyde inorganique dans l'eau et/ou un solvant organique ;
(d) une étape de revêtement de la surface externe des agrégats avec une couche à base de silice, par addition d'une solution aqueuse acide ou alcaline et d'au moins l'un parmi une solution d'un acide silicique, un composé organique du silicium exprimé par la formule chimique (1) ou un produit partiellement hydrolysé de celui-ci, à la dispersion des agrégats des fines particules d'oxyde inorganique :
RₙSi(OR')₄₋ₙ (1)
où R et R' représentent des radicaux hydrocarbonés tels qu'un radical alkyle, un radical aryle, un radical vinyle, un radical acrylique et n est un nombre entier situé dans l'intervalle allant de 0 à 3 ; et
(e) une étape qui consiste à soumettre la dispersion des agrégats de fines particules d'oxyde inorganique revêtus de la couche à base de silice, à
(A) un traitement hydrothermique à une température située dans l'intervalle allant de 50 à 350°C, ou
(B) une séparation et un séchage des fines particules d'oxyde inorganique revêtues de la couche à base de silice, puis chauffage des fines particules d'oxyde inorganique sous pression atmosphérique ou dans un état de dépressurisation, à une température située dans l'intervalle allant de 400 à 1200°C,
et mélanger les particules sphériques et non poreuses à un composant liquide.

10. Procédé de production d'un produit cosmétique selon la revendication 9, où les agrégats de fines particules d'oxyde inorganique sont préparés par pulvérisation d'une solution colloïdale comprenant un hydrogel d'oxyde inorganique et/ou un xérogel avec les fines particules d'oxyde inorganique dans le courant d'air de l'étape (a).
